Europäisches Patentamt

European Patent Office

Office européen des brevets

(19)

(11) **EP 0 903 174 A1**

(12) **EUROPÄISCHE PATENTANMELDUNG**

(43) Veröffentlichungstag:
24.03.1999 Patentblatt 1999/12

(21) Anmeldenummer: 98117121.8

(22) Anmeldetag: 10.09.1998

(51) Int. Cl.$^6$: **B01J 19/00**, C07B 33/00,
C07C 67/42, C07C 69/017,
C07C 315/02

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: 22.09.1997 DE 19741645

(71) Anmelder:
• **BAYER AG**
**51368 Leverkusen (DE)**
• **Forschungszentrum Karlsruhe GmbH**
**76133 Karlsruhe (DE)**

(72) Erfinder:
• Schubert, Klaus Dr.
76227 Karlsruhe (DE)

• **Fichtner, Maximilian Dr.**
**68723 Ofersheim (DE)**
• **Herrmann, Erhard Dr.**
**Club de Golf Chiluca 529 (MX)**
• **Weismantel, Lothar Dr.**
**51065 Köln (DE)**
• **Wiessmeier, Georg Dr.**
**51061 Köln (DE)**
• **Bollyn, Marc Dr.**
**1820 Steenokkerzeel (BE)**

(74) Vertreter: **Drope, Rüdiger, Dr.**
**c/o Bayer AG**
**Konzernbereich RP**
**Patente und Lizenzen**
**51368 Leverkusen (DE)**

(54) **Verfahren und Vorrichtung zur Oxidation organischer Verbindungen in flüssiger Phase unter Verwendung peroxidischer Oxidationsmittel**

(57)     Die organische Verbindung wird in Form einer Lösung durch Zugabe eines peroxidischen, mindestens zwei verbundene Sauerstoffatome (-O - O-) enthaltenden Oxidationsmittels aufoxidiert. Zu diesem Zweck wird ein die organische Verbindung enthaltendes Edukt A und ein das peroxidische Oxidationsmittel enthaltendes Edukt B kontinuierlich miteinander vermischt, dann das flüssige Reaktionsgemisch einem Mikroreaktor (1) mit einer Schar von parallelen Reaktionskanälen (4') und benachbarten Kühlkanälen (3') zugeführt und dabei gleichzeitig auf die Reaktionskanäle (4') verteilt. Die Kühlkanäle (3') werden dabei mit einem Kühlmittel beschickt, um die durch die exotherme Oxidationsreaktion in den Reaktionskanälen (4') erzeugte Wärme im Mikroreaktor (1) abzuführen. Der zu diesem Zweck verwendete Mikroreaktor (1) ist dadurch charakterisiert, daß die größte Kanalabmessung a der Reaktionskanäle (4') senkrecht zur Strömigsrichtung der benachbarten Kanäle <1000 µm, vorzugsweise <500 µm beträgt und die kleinste Wandstärke b zwischen den Reaktionskanälen (4') und den Kühlkanälen (3') <1000 µm, vorzugsweise <100 µm ist. Besonders bewährt hat sich ein Mikroreaktor mit mehreren seriellen Stufen (10a ... 10n), deren Reaktionskanalquerschnitte in Strömungsrichtung zunehmen. Das Verfahren wird bevorzugt zur Oxidation von organischen Sulfiden eingesetzt, wobei als Oxidationsmittel Wasserstoffperoxid verwendet wird.

**Fig. 1**

EP 0 903 174 A1

**Beschreibung**

Die vorliegende Erfindung betrifft ein Verfahren und eine Vorrichtung zur Oxidation organischer Verbindungen in flüssiger Phase, bei dem die organische Verbindung in Form einer Lösung durch Zugabe eines peroxidischen, mindestens zwei verbundene Sauerstoffatome (-O - O-) enthaltenden Oxidationsmittels aufoxidiert wird.

Stoffliche Umsetzungen in chemisch reagierenden Systemen werden begleitet von Reaktionswärmen, die bei exothermen Reaktionen zu Wärmeproduktion führen. Sind die ablaufenden chemischen Reaktionen schnell, so ist die pro Zeiteinheit freigesetzte Reaktionswärme entsprechend groß. Oxidationsreaktionen von organischen Verbindungen in flüssiger Phase, bei denen peroxidische Verbindungen als Oxidationsmittel eingesetzt werden, sind diesem Reaktionstyp zuzuordnen, d. h. sie laufen meist schnell, teilweise explosionsartig und stark exotherm ab.

Unter peroxidischen Verbindungen sind hier chemische Verbindungen zu verstehen, die mindestens eine aus 2 verbundenen Sauerstoffatomen bestehende O-O-Gruppe aufweisen. Diese Verbindungen umfassen sowohl anorganische (z. B. Wasserstoffperoxid, Natriumperborat) als auch organische peroxidische Verbindungen (z. B. Peroxide, Hydroperoxide, Persäuren). Sie werden nachfolgend vereinfacht als Peroxide oder peroxidische Verbindungen bezeichnet.

Eine vordringliche Aufgabe des Reaktionstechnikers besteht im Falle stark exotherm ablaufender chemischer Reaktionen unter Einsatz peroxidischer Verbindungen darin, den Reaktionsablauf temperaturkontrolliert zu führen, d. h. die pro Zeiteinheit freigesetzte Reaktionswärme durch Wärmeübertragung kontrolliert abzuführen, z. B. um die Reaktionstemperatur nach oben zu begrenzen, einen explosionsartigen Verlauf der Reaktion zu verhindern oder um eine gewünschte, für den Prozeß optimale Reaktionstemperatur einzustellen. In vielen Fällen wird dabei eine isotherme Reaktionsführung unter Vermeidung von Temperaturspitzen angestrebt. Temperaturspitzen treten oft zu Beginn der Reaktion aufgrund der hohen Eduktkonzentrationen und, daraus resultierend, aufgrund der hohen Reaktionsgeschwindigkeiten auf. Eine kontrollierte Temperaturführung unter Vermeidung zu hoher Temperaturen ist speziell bei der Durchführung von Reaktionen unter Einsatz von Peroxiden zwingend erforderlich, um die Zersetzung der thermisch empfindlichen und vergleichsweise teuren peroxidischen Verbindungen zu unterdrücken. So liegt beispielsweise die kinetische Aktivierungsenergie der unerwünschten Zerfallsreaktion von Wasserstoffperoxid

$$H_2O_2 \rightarrow H_2O + 0.5\ O_2$$

bei 201 kJ/mol, d. h. hohe Temperaturen führen zu einer starken Beschleunigung der unerwünschten, explosionsartig verlaufenden Zerfallsreaktion. Es ist daher zu gewährleisten, daß das Peroxid nicht oder nur kurzzeitig d. h. im Sekundenbereich, höheren Temperaturen ausgesetzt wird. Weitere Vorteile einer temperaturkontrollierten Reaktionsführung ergeben sich beispielsweise aus der Reduzierung unerwünschter Folgereaktionen des durch Oxidation mit Peroxiden erhaltenen Reaktionsproduktes, speziell wenn die unerwünschte Folgereaktion mit einer höheren Aktivierungsenergie als die gewünschte Hauptreaktion abläuft. Dadurch können Einsatz-und Reststoffe minimiert werden, was zu wesentlich Umwelt- und Ressourcen schonenderen Verfahren führt.

Aus der Anforderung, Reaktionen unter Einsatz peroxidischer Oxidationsmittel temperaturkontrolliert zu führen, ergibt sich die Aufgabe, einen chemischen Reaktor bereitzustellen, der eine ausreichend hohe Wärmeübertragungsleistung aufweist.

Stark exotherm ablaufende Flüssigphasenreaktionen unter Einsatz peroxidischer Verbindungen werden nach dem Stand der Technik meist in Ruhrkesseln bzw. in Reaktionsapparaten mit Rührkesselcharakteristik wie z. B. Schlaufenreaktoren durchgeführt [1]. Dabei ist in der Regel zwischen kontinuierlicher Fahrweise und SemibatchFahrweise zu unterscheiden. Die reine Batch-Fahrweise ist eher unüblich. Kontinuierlich betriebene Rührkessel sind dadurch gekennzeichnet, daß die Konzentrationen der Reaktanden aufgrund der Rückvermischung niedrig sind. Im stationären Zustand und bei idealer Rückvermischung entsprechen die Konzentrationen im Reaktor bekauntermaßen den Konzentrationen am Reaktoraustritt. Dadurch ist der Reaktionsablauf entsprechend langsam und die Wärmeabfuhr kann kontrolliert über die Rührkesselwand oder Wärmetauschereinbauten erfolgen.

Bei Semibatch-Betrieb wird meist eine Reaktionskomponente, in der Regel die peroxidische Verbindung, zu den im Rührkessel vorgelegten Reagentien zudosiert (Zulaufverfahren). Diese Fahrweise ist typisch in Mehrproduktanlagen zur Herstellung von Feinchemikalien [2]. Die Zudosierung des Peroxides erfolgt langsam, meist über viele Stunden, so daß die bei der chemischen Reaktion freiwerdende Reaktionswärme kontrolliert über die Wärmeübertragerflächen abgeführt und eine definierte, nicht zu hohe Temperatur im Rührkessel eingestellt werden kann.

Nachteilig bei der konventionellen Konti- bzw. Semibatch-Fahrweise ist die geringe Reaktorleistung, d. h. die geringe Produktionsmenge pro Volumen- und Zeiteinheit. Daraus resultieren in vielen Fällen unerwünscht große Reaktoren oder eine Vielzahl parallel geschalteter, höhere Investitions- und Betriebskosten verursachende Einheiten. Weitere Nachteile ergeben sich aus Ausbeute- und Selektivitätseinbußen aufgrund der langen Reaktorverweilzeiten und aufgrund der breiten Verweilzeitspektren. Da bei stark exothermen Flüssigphaserreaktionen unter Einsatz von Peroxiden große Wärmemengen abgeführt werden müssen, muß das Kühlmittel oft auf sehr niedriger Temperatur gehalten werden, so daß größere Temperaturgradienten zwischen Rührkesselvolumen und Rührkesselwand auftreten. Dies fördert die Bildung von Ablagerungen auf den Wärmeübertragerflächen, z. B. infolge von Kristallisation und erhöht dadurch den erforderlichen Reinigungsaufwand. Der Scaleup, d. h. die Maßstabsvergrößerung eines Rührkessels, im Falle einer Verfahrensentwicklung oder bei Erhöhung der Produktionskapazität, wird weiterhin dadurch erschwert, daß das Verhältnis von Wärmeübertragerfläche zu Rührkesselvolumen mit zunehmender Rührkesselgröße kleiner wird. Oft behilft man sich in solchen Fällen damit, daß das Reaktionsgemisch in einem äußeren Flüssigkeitskreislauf umgepumpt wird und ein großer Teil der Reaktionswärme in einem im Kreislauf angeordneten Wärmeübertrager abgeführt wird.

Die genannten Nachteile lassen sich bei Flüssigphasenoxidationen organischer Verbindungen unter Einsatz von Peroxiden als Oxidationsmittel dadurch vermeiden oder zumindest reduzieren, daß die Reaktion kontinuierlich und weitgehend rückvermischungsfrei im Strömungsrohr oder Reaktoren mit Strömungsrohrcharakteristik durchgeführt wird. Reaktionen können darin bekanntermaßen, je nach zugrundeliegendem Reaktionsschema, mit höherer Ausbeute und Selektivität, z. B. im Falle partiell ablaufender Oxidationen, in kurzen Reaktionszeiten und folglich mit großen Raum-Zeit-Ausbeuten durchgeführt werden. Aus sicherheitstechnischer Sicht ist der geringe Holdup in solchen Reaktoren vorteilhaft. Die technische Durchfürung stark exotherm ablaufender Flüssigphasenreaktionen mit Peroxiden in konventionellen Reaktoren mit Strömungsrohrcharakteristik, wie z. B. Doppelrohr oder Rohrbündelreaktor, scheitert bislang daran, daß die Reaktion aufgrund des höheren Konzentrationsniveaus der Reaktionskomponenten deutlich schneller als in Rührkesseln abläuft und damit die pro Zeiteinheit frei werdende Reaktionswarme in vielen Fällen nicht mehr sicher und kontrolliert abgeführt werden kann. Die unzureichende Wärmeabfuhr führt zu Überhitzung und im Extremfall zu einer explosionsartigen Zersetzung der thermisch empfindlichen Peroxide.

Zur Nutzung des für Reaktoren mit Strömungsrohrcharakteristik beschriebenen Verbesserungspotentials bei der Durchführung von Flüssigphasenoxidationen mit peroxidischen Verbindungen als Oxidationsmittel besteht eine wesentliche Aufgabe darin, neuartige Reaktorsysteme einzusetzen, die eine um mindestens den Faktor 10 bis 100 höhere Wärmeübertragungsleistung als konventionelle Reaktoren aufweisen.

Diese Aufgabe wird erfindungsgemäß dadurch gelöst, daß ein die organische Verbindung enthaltender Eduktstrom A und ein das peroxidische Oxidationsmittel enthaltender Eduktstrom B kontinuierlich miteinander vermischt werden und das flüssige Reaktionsgemisch einem Mikroreaktor mit einer Schar von parallelen Reaktionskanälen und benachbarten Kühlkanälen zugeführt und dabei auf die Reaktionskanäle verteilt wird. Gleichzeitig werden dabei die Kühlkanäle mit einem Kühlmittel beschickt, um die durch die exotherme Oxidationsreaktion in den Reaktionskanälen erzeugte Wärme im Mikroreaktor abzuführen. Der zu diesem Zweck eingesetzte Mikroreaktor ist dadurch charakterisiert, daß die größte Kanalabmessung a der Reaktionskanäle serikrecht zur Strömungsrichtung der benachbarten Kanäle <1000 $\mu$m, vorzugsweise <500 $\mu$m beträgt und die kleinste Wandstärke b zwischen den Reaktionskanälen und den Kühlkanälen <1000 $\mu$m, vorzugsweise <100 $\mu$m ist.

Vorteilhaft weisen dabei die Reaktionskanäle einen hydraulischen Kanaldurchmesser d <1000 $\mu$m, vorzugsweise <500 $\mu$m, auf.

Vorzugsweise wird dieses Verfahren zur Oxidation organischer Sulfide eingesetzt; d.h. die zu oxidierende Verbindung besteht aus einem organischen Sulfid.

Besonders bewährt hat sich das erfindungsgemäße Verfahren zur Oxidation von 3,5-Dithio-heptane-1,7-diol-diacetat. Dabei wird als peroxidisches Oxidationsmittel Wasserstoffperoxid verwendet.

Als weitere zum Einsatz des erfindungsgemäßen Verfahren besonders geeignete Reaktionen haben sich die oxidative Spaltung von Olefinen zu Carbonsäuren unter Verwendung von Wasserstoffperoxid, insbesondere mit Natriumwolframat als Kata-lysator, die Oxidation von Ketonen zu Carbonsäureestern nach Baeyer-Villiger und die Oxidation von Thiolen zu Sulfonsäuren unter Verwendung von Wasserstoffperoxid als Oxidationsmittel erwiesen.

Zur Erzeugung des die organische Verbindung und das peroxidische Oxidationsmittel enthaltenden Reaktionsgemisches wird vorteilhaft ein Düsenmischer verwendet, bei dem das eine Edukt in das andere eingedüst wird.

Gemäß einer besonderen Ausführungsform wird das Reaktionsgemisch durch einen dem Mikroreaktor vorgeschalteten Rohrreaktor mit einer Verweilzeit von 10 ms bis 20s, vorzugsweise von 10 ms bis 10 s gefördert. Dabei kann man von der vorteilhaften Variante Gebrauch machen, daß die Reaktion im Rohrreaktor unter adiabatischen Bedingungen durchgeführt wird und das heiße Reaktionsgemisch im Mikroreaktor innerhalb von 1 ms bis 10 s, vorzugsweise innerhalb von 1 ms bis 1 s, um mehr als 20°C heruntergekühlt wird. Auf diese Weise kann eine erhebliche Selektivitätssteigerung erreicht werden.

Gemaß einer Weiterentwicklung des erfindungsgemäßen Verfahrens wird das Reaktionsgemisch durch mehrere seriell hintereinander geschaltete Mikroreaktoren gefördert. Vorteilhaft nimmt dabei der Reaktionskanalquerschnitt in Strömungsrichtung zu.

Dabei besteht die Möglichkeit, daß zwischen den Mikroreaktoren oder zwischen dem Düsenmischer und dem Mikroreaktor an einer oder mehreren Stellen Edukte A, B oder im Kreislauf geführtes Reaktionsgemisch eingespeist werden.

Eine zur Durchführung des erfindungsgemäßen Verfahrens geeignete Vorrichtung besteht aus einem Mischer zur kontinuierlichen Vermischung mindestens zweier Eduktsröme A,B und einem nachgeschalteten Mikroreaktor mit Reaktionskanälen und Kühlkanälen, wobei die größte Kanalabmessung a der Reaktionskanäle senkrecht zur Strömungsrichtung der benachbarten Kanäle <1000 µm, vorzugsweise <500 µm beträgt und die kleinste Wandstärke b zwischen den Reaktionskanälen und den Kühlkanälen <1000 µm, vorzugsweise <100 µm ist. Erfindungsgemäß ist dabei der Mikroreaktor seriell in mindestens zwei Mikroreaktorstufen unterteilt, deren Reaktionskanalquerschnitte in Strömungsrichtung stufenweise zunimmt.

Gemäß einer bevorzugten Ausführung besitzen die Reaktionskanäle der seriell hintereinander geschalteten Reaktorstufen einen hydraulischen Kanaldurchmesser d <1000 µm, vorzugsweise <500 µm.

Vorteilhaft ist dabei eine Kreislaufführung für das Reaktionsgemisch oder die Zufuhr von frischem Edukt zwischen einer oder mehreren Mikroreaktorstufen.

Gemäß einer bevorzugten Ausführungsform ist zwischen dem Mischer und den Mikroreaktorstufen ein adiabatischer Rohrreaktor angeordnet.

Mit der Erfindung werden folgende Vorteile gegenüber der konventionellen Rührkessel-Fahrweise erzielt:

Aufgrund der kontrollierten Temperaturführung sowie aufgrund der kurzen Verweilzeiten des Reaktionsgemisches im Mikroreaktor lassen sich höhere Ausbeuten bei Flüssigphasenreaktionen erzielen. Die Verluste durch Zersetzung des peroxidischen Oxidationsmittels werden reduziert. Die Raum-Zeit-Ausbeuten lassen sich gegenüber konventionellen Fahrweisen deutlich erhöhen. So können die Reaktionslaufzeiten zur Herstellung einer bestimmten Produktmenge bei Einsatz von kontinuierlich beschickten Mikroreaktoren von Stunden auf Minuten verringert werden. Weiterhin kann der Reinigungsaufwand reduziert werden, z. B. durch einfaches Durchspülen der Apparatur. Daraus und aus der Möglichkeit einer einfacheren Automatisierung des Verfahrensablaufes können die Herstellkosten stark reduziert werden. Weitere Vorteile resultieren aus höheren, reproduzierbaren Produktqualitäten und dem geringeren sicherheitstechnisch relevanten Reaktor-Holdup.

Aufgrund der großen Wärmeübergangskoeffizienten im Mikroreaktor kann in vielen Anwendungsfällen anstelle eines aufwendigen Kältekreislaufs (z. B. Sole, Ammoniak, Frigen) ein Kühlwasserkreislauf eingesetzt werden. Dies führt zu Einsparungen beim Energiebedarf. Aufgrund der geringen Baugröße des Mikroreaktors und der relativ niedrigen Anschaffungskosten im Vergleich zu Rührkesselreaktoren können mehrere Mikroreaktoren verschaltet werden, z. B. zur Erhöhung der Durchsatzmengen, oder durch unterschiedlich aufgebaute bzw. neuwertige Mikroreaktoren ausgetauscht werden. Vorstellbar ist auch, daß die Mikroreaktoren im Sinne von „Wegwertreaktoren" nach einer bestimmten Einsatzdauer ersetzt werden.

Im Folgenden wird die Erfindung an Hand von Zeichnungen und Ausführungsbeispielen näher beschrieben.

Es zeigen

Fig. 1          den prinzipiellen Aufbau eines Mikroreaktors

Fig. 2          ein schematisches Fließbild für eine Anlage zur Durchführung des erfindungsgemäßen Verfahrens

Fig. 3          einen Querschnitt durch einen Düsenmischer

Fig. 4a-4c      verschiedene Ausführungen von seriell hintereinander geschalteten Mikroreaktorstufen und

Fig.5           zwei hinteinandergeschaltete Mikroreaktoren mit einem dazwischen geschalteten Rohrreaktor

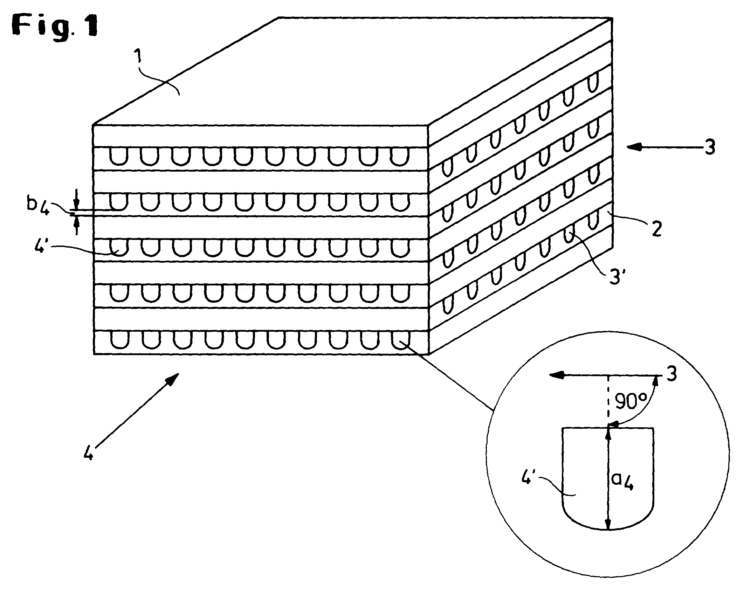

Mikrowärmetauscher sind z. B. in [3] (DE 37 09 278) oder in [4] (US 4 516 632) beschrieben. In [5] ist die Verwendung solcher oder ähnlich aufgebauter Mikrowärmeübertrager als chemische Mikroreaktoren in allgemeiner Form beschrieben. Zur Erläuterung der Funktionsweise eines solchen Mikrowärmeübertragers zeigt Fig. 1 beispielhaft den Aufbau des in [3] beschriebenen Mikrowärmeübertragers 1. Dieser besteht aus einem Stapel diffusionsverschweißter Metallfolien 2 mit Foliendicken von z. B. 100 µm. In diese Metallfolien werden mit Hilfe formgeschliffener Werkzeuge parallel zueinander verlaufende Mikrokanäle 4' für eine Reaktionsmischung 4 und Mikrokanäle 3' für ein Kühlmittel 3 eingebracht. Die minimal realisierbaren Kanalabmessungen liegen bei [3] im Bereich von 10 µm. Die geometrische Form der Mikrokanäle 3' und 4' ist frei wählbar. So sind z. B. Rechteck- wie auch kreisförmige Querschnitte möglich. Die Mikrokanäle 3'und 4' können unterschiedliche Abmessungen aufweisen. Um gleiche Durchflußmengenströme in den einzelnen Mikrokanälen einer Fluidpassage zu gewährleisten, sind die Mikrokanäle einer Fluidpassage untereinander gleich. Der charakteristische hydraulische Kanaldurchmesser von Mikrokanälen der Fluidpassage i (hier: i=3 bzw. 4) ergibt sich aus der Beziehung

$$d_i = 4\,A_i/U_i \text{ , wobei}$$

$d_i =$          charakteristischer hydraulischer Kanaldurchmesser der Fluidpassage i

$A_i =$          durchströmter Kanalquerschnitt der Fluidpassage i

$U_i =$          benetzter Kanalumfang der Fluidpassage i

$i =$           Index für die Fluidpassage (Anzahl der Fluidpassagen $\geq 2$)

Fig. 1 zeigt ebenfalls einen als vergrößerten Ausschnitt dargestellten Mikrokanal 4' der Fluidpassage 4 mit der Kanalabmessung $a_4$, wobei $a_4$ die größte Abmessung des Mikrokanals 4' senkrecht zur Strömungsrichtung der Kühlmittelpassage 3 ist. Weiterhin ist die kleinste Wandstärke $b_4$, d. h. der geringste Abstand zwischen den beiden Fluidpassagen 3 und 4 eingezeichnet.

Mikroreaktoren sind hier allgemein dadurch gekennzeichnet, daß entweder

- die charakteristischen hydraulischen Kanaldurchmesser $d_i$ (hier: i=3 und 4) oder
- die Kanalabmessung $a_i$ (in den Reaktionskanälen)

aller Mikrokanäle zumindest einer Fluidpassage i Kleiner 1000 $\mu$m sind. Die kleinste Wandstärke $b_i$ zwischen den einzelnen Fluidpassagen ist ebenfalls kleiner 1000 $\mu$m, vorzugsweise kleiner 100 $\mu$m zu wählen. Diese Aussagen gelten auch für den Fall, daß die Mikrokanäle einer Fluidpassage i untereinander unterschiedlich groß sind.

Die einzelnen Metallfolien werden im Beispiel von Fig. 1 so übereinander gestapelt, daß die Mikrokanäle zweier benachbarter Fluidpassagen unter 90° zueinander verlaufen (Kreuzstrom-Mikrowarmeübertrager) und heliumdicht gegeneinander abgedichtet sind. Bei der Nutzung des Mikrowärmeübertragers als chemischer Mikroreaktor wird eine Fluidpassage für die Strömungsführung des Reaktionsgemisches 4, die andere Fluidpassage für die Strömungsführug des Kühlmittels 3 verwendet. Neben der in Fig. 1 dargestellten Kreuzstromführung sind aber auch andere typische Strömungsführungen wie Gleich- und Gegenstromführung und alle Kombinationen daraus realisierbar. Die deutliche Erhöhung der Wärmeübertragungsleistung im Mikroreaktor beruht darauf, daß durch die kleinen hydraulischen Kanaldurchmesser $d_i$, vor allem aber durch die Kleinen Kanalabmessungen $a_i$, die Transportwege für die zwischen den Fluidpassagen zu übertragenden Wärmeströme sehr kurz sind. Gegenüber Wärmedurchgangskoeffizienten von ca. 1000 W/m$^2$ K in konventionellen Reaktoren (Flüssigkeit auf der Reaktions- und Kühlmittelseite) ergeben sich in Mikroreaktoren Werte in der Größenordnung 20000 W/m$^2$ K (beide Fluidpassagen: $d_i$ = 80 $\mu$m, $a_i$ = 100 $\mu$m, Wasser). Die spezifische Wärmeübertragerfläche kann Werte größer 100 cm$^2$/cm$^3$ erreichen gegenüber ca. 1 cm$^2$/cm$^3$ in konventionellen Rohrbündelreaktoren. Daraus resultiert insgesamt eine Steigerung der volumenspezifischen Wärmeübertragungsleistung um mindestens einen Faktor 1000.

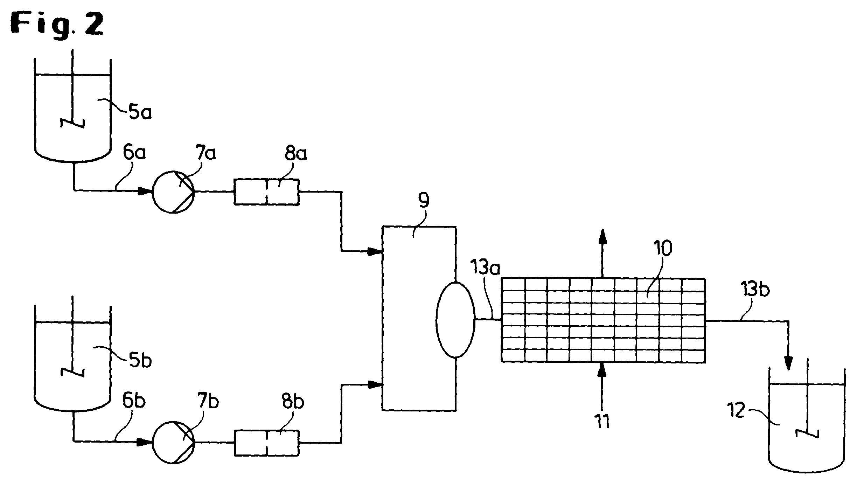

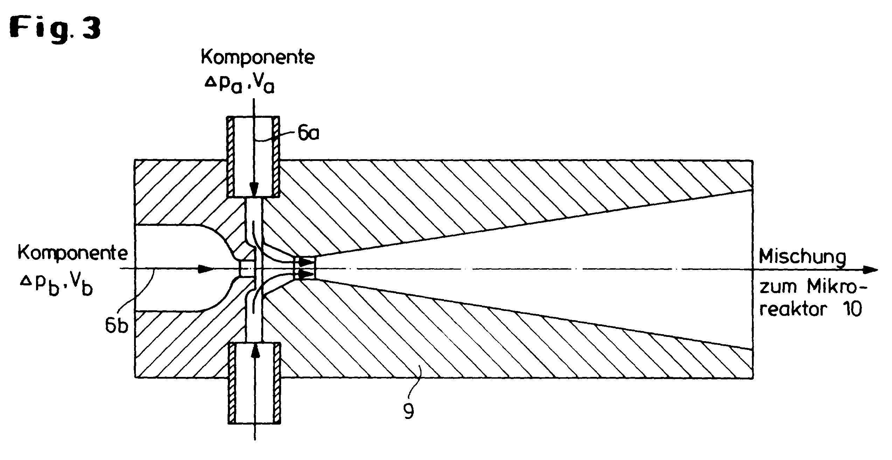

Fig. 2 zeigt eine bevorzugte Anlage zur Durchführung stark exothermer Flüssigphasenreaktionen mit Peroxiden als Oxidationsmittel und unter Einsatz von Mikroreaktoren zur Abfuhr der Reaktionswärme. Mindestens zwei Eduktströme 6a und 6b (im allgemeinen Fall m Eduktströme 6a bis 6m) werden aus getrennten Eduktvorlagebehältern 5a und 5b über Förderorgane 7a und 7b sowie wahlweise über Filterelemente 8a und 8b einem Mischaggregat 9 zugeführt. Zumindest einer der Eduktströme enthält die peroxidische Verbindung bzw. eine Vorläuferform, aus der durch Reaktion in situ die peroxidische Verbindung entsteht. Zur Vermischung der Reaktionskomponenten werden vorzugsweise schnell mischende Apparate eingesetzt, beispielsweise der in Fig. 3 dargestellte Düsenmischer 9. Der Zulauf von 6a und 6b kann auch vertauscht sein. Die Misclizeiten solcher Düsenmischer liegen in der Größenordnung von Millisekunden. Nach erfolgter Vermischung tritt das Reaktionsgemisch unmittelbar in den Mikroreaktor 10 ein. Damit ein möglichst großer Teil der Reaktion temperaturkontrolliert unter Kühlung mit einem Kühlmittel 11 abläuft bzw. keine zu hohen Temperaturen vor Eintritt in den Mikroreaktor auftreten, muß die Verweilzeit zwischen Mischaggregat 9 und Mikroreaktor 10 klein gewählt werden. Auf eine gleichmäßige Anströmung des Mikroreaktors ist zu achten. Die Verweilzeit im Mikroreaktor 10 liegt typischerweise im Bereich zwischen 1 ms und 20 s.

Eine Variante sieht vor, daß zwischen Mischaggregat 9 und Mikroreaktor 10 eine Rohrstrecke 13a mit einer Verweilzeit von 10 ms bis 20 s, vorzugsweise von 10 ms bis 10 s , geschaltet wird, damit sich das Reaktionsgemisch aufgrund der freigesetzten Reaktionswärme vor Eintritt in den Mikroreaktor 10 selbsttätig auf Reaktionstemperatur erwärmt. Eine weitere aus Fig. 2 ableitbare Variante besteht darin, daß eine bestimmte Anzahl von Mikroreaktoren zur Erhöhung der Durchsatzleistung parallel verschaltet wird. Nach erfolgter Reaktion wird das aus 10 austretende Produktgemisch über eine weitere Rohrstrecke 13b einem Produktauffangbehälter 12 zugeführt. Optional können dem Mikroreaktor 10 auch ein oder mehrere konventionelle Wärmeübertrager zur Abfuhr von Restwärme nachgeschaltet werden. Durch kontinuierliches Befüllen der Eduktvorlagebehälter und Entleeren der Produktauffangbehälter kann im Bedarfsfall ein kontinuierlicher Betrieb der Apparatur auch über längere Zeiträume aufrechterhalten werden. Ein kontinuierlicher Betrieb ist auch realisierbar durch den Einsatz eines kontinuierlich arbeitenden Bandfilters zur Produktaufbereitung anstelle des Auffangbehälters 12.

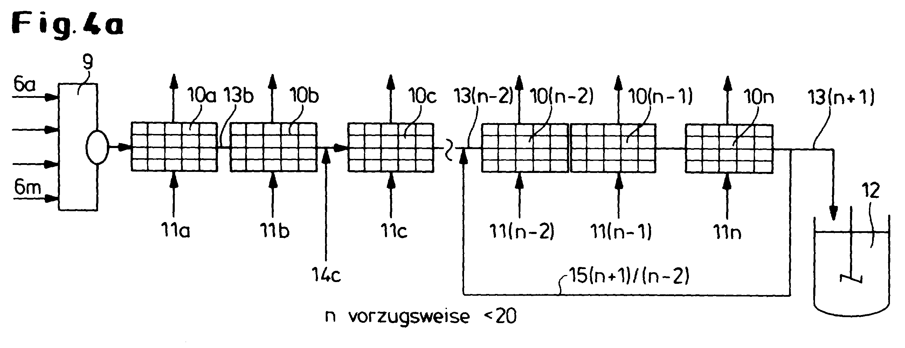

Durch serielle Verschaltung von zwei oder mehreren, wegen des erforderlichen Verschaltungsaufwandes aber vorzugsweise von kleiner 20, gleich oder unterschiedlich aufgebauten Mikroreaktoren kann eine hinsichtlich Ausbeute und Selektivität der Reaktion besonders vorteilhafte Temperaturführung realisiert werden. Fig. 4a zeigt die serielle Verschaltung mehrerer Mikroreaktoren 10a bis 10n, wobei die einzelnen Mikroreaktoren wahlweise ohne Zwischerräume, also direkt oder über mehrere Rohrstrecken 13a bis 13n miteinander verbunden sind. Allgemein gilt, daß dem letzten Mikroreaktor 10n einer Kaskade konventionelle Wärmeübertrager nachgeschaltet werden können. Die Kühlmittelströme 11a bis 11n können seriell und/oder parallel miteinander verschaltet werden. Typischerweise wird ein Kühlmittelkreislauf installiert, wobei die Kühlmittelströme 11a bis 11n seriell miteinander verknüpft werden. Alternativ können mehrere, unabhängig voneinander betriebene Kühlmittelkreisläufe betrieben werden. Neben der hier dargestellten Kreuzstromführung des Wärmeträgers innerhalb des Mikroreaktors kann auch eine Gegenstrom-, Kreuzgegenstrom-, Gleichstrom- oder Kreuzgleichstromführung sowie alle Kombinationen daraus realisiert werden. Zur Einstellung gleicher Temperaturprofile längs der Mikrokanäle einer Fluidpassage ist oft eine Gleich- bzw. Gegenstromführung der Kreuzstromführung vorzuziehen.

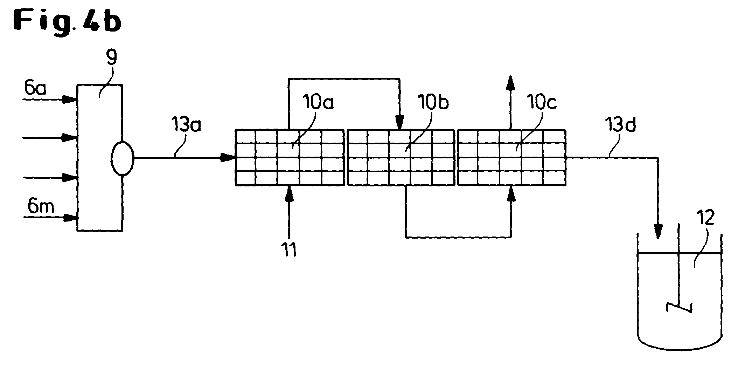

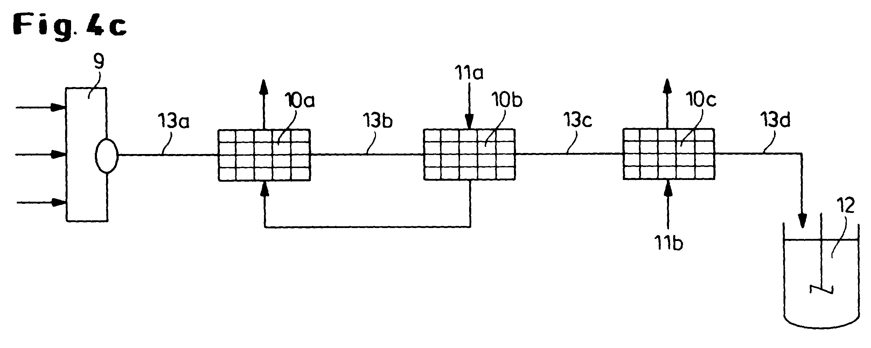

In Fig. 4b und Fig. 4c sind zur Veranschaulichung zwei von mehreren möglichen Verschaltungsvarianten des Kühlmittels dargestellt. Fig. 4b zeigt eine Kreuzgleichstromführung mit einem Kühlmittelkreislauf 11, bei der die Mikroreaktoren 10a bis 10c direkt miteinander verbunden sind. Fig 4c illustriert eine Kreuzgegenstromführung des Kühlmittels 11a sowie eine einfache Kreuzstromführung des Kühlmittels 11b in einem zweiten Kühlkreislauf. Die einzelnen Mikroreaktoren 10a bis 10c sind über ungekühlte Rohrstrecken 13a bis 13c miteinander verbunden. Im Bereich der ungekühlten zwischengeschalteten Rohrstrecken 13a bis 13c in Fig. 4c läuft die Reaktion näherungsweise adiabat unter Temperaturanstieg ab. Das Reaktionsgemisch wird nach jeder Rohrstrecke im Mikroreaktor heruntergekühlt, sodaß insgesamt ein sägezahnartiger Temperaturverlauf resultiert. Der Vorteil einer solchen Schaltung besteht darin, daß das erforderliche Mikroreaktorvolumen reduziert werden kann, was vor allem bei Reaktionen mit Reaktionszeiten im Bereich größer ca. 3 Sekunden vorteilhaft ist. Die durch Wandreibung in den Mikrokanälen des Mikroreaktors verursachten Druckverluste, vor allem im Fall höherviskoser Fluide, werden bei einer solchen Verschaltung reduziert.

Zur weiteren Reduzierung der Druckverluste können die Mikrokanalabmessungen an die sich im Laufe der chemischen Umsetzung ändernde chemische Wärmeproduktion angepaßt werden. In der Regel laufen chemische Reaktionen in der Weise ab, daß die chemische Reaktion zu Beginn schnell ist und eine hohe Wärmeproduktion pro Zeiteinheit aufweist. Dementsprechend müssen die Mikrokanalabmessungen zu Beginn der Reaktion klein sein, um eine ausreichend große Wärmeübertragungsleistung zu gewährleisten. Mit fortschreitendem Umsatz wird die Reaktion in der Regel langsamer und die Mikrokanalabmessungen können entsprechend größer gewählt werden. Die daraus resultierende apparative Verschaltung erfolgt entsprechend Fig. 4a bis 4c, wobei die Kanalabmessungen der einzelnen Mikroreaktoren in Strömungsrichtung des Reaktionsgemisches größer werden.

Zwischen den einzelnen Mikroreaktoren in Fig. 4a können an einer oder mehreren Stellen wahlweise frische Eduktlösungen 14j (j=a bis n) oder im Kreislauf geführtes Reaktionsgemisch bzw. Produktlösung 15 ($j_1$/$j_2$) ($j_1$ = b bis (n+1), $j_2$ = a bis n) eingespeist werden. So kann beispielsweise ein Teil der benötigten Peroxidmenge über den Düsenmischer, der Rest als Teilstrom 14c zwischen den Mikroreaktoren 10b und 10c zudosiert werden. Dies hat den Vorteil, daß die Peroxidkonzentration im Reaktionsgemisch gering gehalten und eine Zersetzung des Peroxids zurückgedrängt wird.

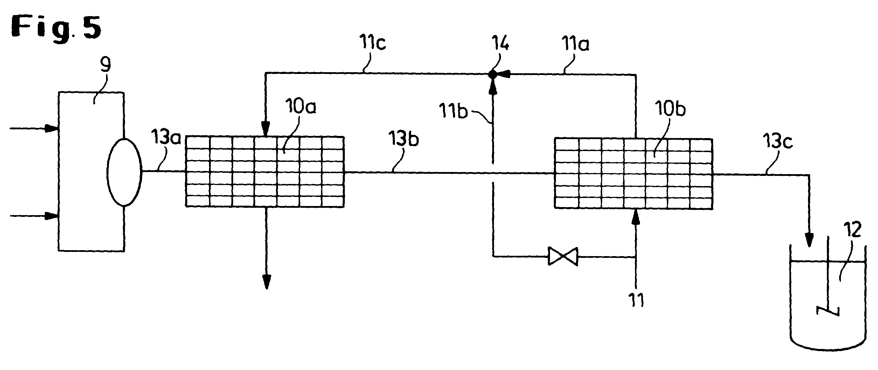

Eine bevorzugte Verschaltung der Mikroreaktoren zeigt Fig. 5. Zwei in Serie geschaltete Mikroreaktoren 10a und 10b sind durch eine Rohrstrecke 13b voneinander getrennt. Im ersten Mikroreaktor 10a wird die unmittelbar nach der Vermischung in 13a, sowie die in 10a freigesetzte Reaktionswärme kontrolliert abgeführt. Hierbei gewährleistet der Mikroreaktor 10a eine kontrollierte und selektive Reaktionsführung zu Beginn der Reaktion. Dies ist gerade bei Reaktionsbeginn von Bedeutung, da hier noch hohe Eduktkonzentrationen und damit verbunden eine hohe Reaktivität vorliegen. Der Resturnsatz mit einer in der Regel geringeren Wärmeproduktion pro Zeiteinheit erfolgt in der nachgeschalteten Rohrstrecke 13b. Die dabei freigesetzte Restwärme wird in einem zweiten Mikroreaktor 10b abgeführt. Der zweite Mikroreaktor 10b hat in diesem Fall die Zusatzfunktion eines Sicherheitswärmeübertragers, der das Reaktionsgemisch auf niedrige Temperaturen abkühlt, sodaß nach dieser Einheit keine nennenswerten Reaktionsumsätze mehr erfolgen können, d. h. eine nennenswerte Reaktion in der nachgeschalteten Produktvorlage 12 nicht auftreten kann. Optional kann durch eine Bypassschaltung ein Teil des Kühlmittels 11a durch den Mikroreaktor 10b, der andere Teil 11b im Bypass am Mikroreaktor 10b vorbei geführt werden. Im Mischpunkt 14 werden der vorgewärmte Kühlmittelstrom 11a und der kalte Strom 11b zusammengeführt. Dabei kann die Temperatur des Kühlmittelstroms 11c variabel eingestellt werden.

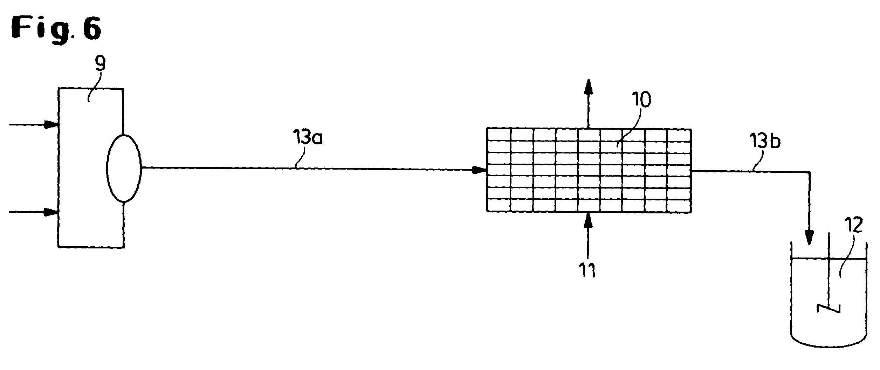

Eine weitere realisierte Reaktionsführung zeigt Fig. 6. Nach Vermischung der Reaktanden im Mischaggregat 9 erfolgt die Reaktion zunächst adiabat in einer ungekühlten Rohrstrecke 13a. Nach erfolgter vollständiger Umsetzung oder Teilumsetzung der Reaktanden wird das heiße Reaktionsgemisch in einem Mikroreaktor 10 spontan innerhalb von Millisekunden auf niedrigere Temperaturen heruntergekühlt. Die Reaktion kann dabei in 10 weiterlaufen oder kommt beim Herunterkühlen zum Stillstand. Damit können unerwünschte Nebenreaktionen wie z. B. die thermisch bedingte Zersetzung des Peroxides oder andere Folgereaktionen unterdrückt werden. Typisch ist eine Reaktionsführungsvariante, bei der die Reaktion bis zu einem Teilumsatz, bei dem eine hohe Selektivität erreicht wird, in der Rohrstrecke 13a abläuft und anschließend durch spontanes Kühlen im Mikroreaktor 10 abgestoppt wird.

Neben der beschriebenen Flüssigphasenoxidation unter Einsatz von Peroxiden als Oxidationsmittel eignen sich die in Fig. 2 bis 6 beschriebenen Vorrichtungen standardmäßig auch zur Durchührung anderer schnell ablaufender, stark exothermer Flüssig- und Gasphasenreaktionen.

Anwendungsfelder des hier beschriebenen Verfahrens und der in Fig. 2 bis 6 gezeigten Vorrichtungen liegen zum einen im Bereich der chemischen Produktion, zum anderen aber auch im Bereich der Verfahrensentwicklung (verfahrensoptimierung, Parameterstudien, kinetische Untersuchungen, Entwicklung und Screening neuer Verfahrenkonzepte).

## Ausführungsbeispiele

### Beispiel 1

In einer Versuchsapparatur entsprechend Fig 2 wurde die homogen katalysierte Flüssigphasenoxidation des organischen Disulfids 3,5-Dithio-heptan-1,7 diol-diacetat zum entsprechenden Disulfon unter Einsatz von Wasserstoffperoxid als Oxidationsmittel und unter Einsatz eines Mikroreaktors untersucht.

Für diese Reaktion ergibt sich folgende Bruttoreaktionsgleichung:

$$CH_3\text{-}CO\text{-}O\text{-}CH_2\text{-}CH_2\text{-}S\text{-}CH_2\text{-}S\text{-}CH_2\text{-}CH_2\text{-}O\text{-}CO\text{-}CH_3 + 4\ H_2O_2 \rightarrow CH_3\text{-}CO\text{-}O\text{-}CH_2\text{-}CH_2\text{-}SO_2\text{-}CH_2\text{-}SO_2\text{-}CH_2\text{-}CH_2\text{-}O\text{-}CO\text{-}CH_3 + 4\ H_2O$$

Die stark exotherm und schnell ablaufende Reaktion läuft dabei über die Sulfoxidbzw. Disulfoxidstufe zum Sulfon bzw. Disulfon ab. Die Reaktionswärme pro mol Formelumsatz beträgt 1100 kJ. Als Homogenkatalysator wird Natriumwolframat eingesetzt.

Das Hauptproblem bei der konventionell in Semibatch-Fahrweise durchgeführten Reaktion besteht in der Wärmeabfuhr (s. auch [1]). Dabei kann das Oxidationsmittel $H_2O_2$ nur langsam zu der vorgelegten Menge an Disulfid zudosiert werden. Für die Herstellung von Produktlösung im $m^3$-Maßstab ist dabei, je nach Größe des verwendeten Ruhrkessels, eine Dosierdauer von größer 20 Stunden erforderlich, was einen verhältnismäßig hohen Zeit-, Personal- und Sicherheitsaufwand erfordert. Die Ausbeute an Disulfon, bezogen auf eingesetztes Disulfid, beträgt 75 %.

Bei der kontinuierlichen Beschickung des Mikroreaktors entsprechend Fig. 2 werden in der Vorlage 5a 3,79 kg des Disulfids mit 15,75 kg Essigsäure bei 20°C vorgelegt. In der Vorlage 5b werden 6,4 kg 35 %ige Wasserstoffperoxidlösung mit 299 g Wasser und 99 g Natriumwolframat bei ebenfalls 20 °C vorgelegt. Über zwei Zahnrad-pumpen 7a und 7b werden die beiden Eduktströme 6a (Disulfid-Lösung) und 6b (Wasserstoffperoxid-Lösung) im Verhältnis 2,8:1 dem Mischaggregat 9 entsprechend Fig. 3 zugeführt. Unmittelbar nach der Vermischung in 9 startet die Reaktion. Der Gesamtdurchsatz beträgt 36,7 kg/h. Die Verweilzeit in der ungekühlten Rohrstrecke 13a zwischen Mischer 9 und Mikroreaktor 10 beträgt 3,3 Sekunden. Dabei erwärmt sich das Gemisch von 20°C auf 113°C unmittelbar am Eintritt in den Mikroreaktor 10. Der Einsatz einer dem Mikroreaktor vorgeschalteten Rohrstrecke hat den Vorteil, daß das Reaktionsgemisch selbsttätig auf höhere Reaktionstemperaturen erwärmt wird. Der eingesetzte Mikroreaktor weist eine Kreuzstromführung bezüglich Reaktions- und Kühlmittelpassage auf. Die Verweilzeit des Reaktionsgemisches im Mikroreaktor beträgt 1,1 Sekunden. Die Austrittstemperatur des Reaktionsgemisches aus dem Mikroreaktor beträgt 37°C. Im Kreuzstrom zum Reaktionsgemisch werden 34,2 kg/h des Kühlwassers 11 durch den Mikroreaktor geleitet. Die Kühlwassereintrittstemperatur beträgt 12°C, die Austrittstemperatur 56°C. Die Verweilzeit des Kühlwassers im Mikroreaktor liegt bei 1,2 Sekunden. Der hydraulische Kanaldurchmesser d entsprechend G1. 1 beträgt für beide Fluidpassagen 160 μm, die Kanalabmessungen a entsprechend Fig. 1 senkrecht zur Strömungsrichtung der benachbarten Fluidpassage jeweils 140 μm. Nach Austritt aus dem Mikroreaktor durchläuft das Reaktionsgemisch eine ungekühlte Rohrstrecke 13b bis zum Einlauf in den Produktvorlagebehälter 12. Die Verweilzeit zwischen Mikroreaktor 10 und Eintritt in die Produktvorlage 12 beträgt 24,5 Sekunden. Dabei erwärmt sich das Reaktionsgemisch von 37°C auf 49°C aufgrund des Restumsatzes. Anschließend wird das Produktgemisch in die Produktvorlage 12 geleitet und für die Analyse vorbereitet.

Die auf diesem Weg erzielte Produktausbeute an Disulfon bezogen auf eingesetztes Disulfid beträgt 76,2 % bei sehr guter Produktqualität. Gegenüber konventioneller Semibatch-Fahrweise kann die Raum-Zeit-Ausbeute um einen Faktor größer 4 gesteigert werden.

### Beispiel 2

Bei einer gegenüber Beispiel 1 modifizierten Fahrweise werden 99 g Natriumwolframat mit 299 g Wasser in einem separaten dritten Eduktvorlagebehälter (nicht gezeichnet) angesetzt und unmittelbar vor dem Mischer 9 mit der 35%igen Wasserstoffperoxid-Lösung 6b in einem Verhältnis 0.06:1 kontinuierlich vermischt. Dies hat gegenüber der oben beschriebenen Fahrweise den Vorteil, daß im Vorlagebehälter 5b keine Zersetzung des dort vorgelegten Wasserstoffperoxides unter Einwirkung von Natriumwolframat auftritt. Die erzielte Ausbeute an Disulfon entspricht der in Beispiel 1.

### Beispiel 3

In einer Versuchsapparatur entsprechend Fig. 4a mit zwei Mikrostruktur-Wärmeübertragern in Serie geschaltet, einer Rohrstrecke zwischen den Wärmeübertragern 13b von 144 cm und parallel geschalteten Kühlströmen wurde die homogen katalysierte Flüssigphasenoxidation des organischen Disulfids 3,5-Dithio-heptan-1,7-diol-diacetat zum entsprechenden Disulfon durchgeführt.

Bei der kontinuierlichen Beschickung des Mikroreaktors werden 5,33 kg Disulfid gelöst in 8,76 kg Essigsäure (Eduktstrom 6a) und 5,13 kg Wasserstoffperoxid (35%ig), 40 g Natriumwolframat und 119 g Wasser (Eduktstrom 6b) im Mischaggregat 9 im Verhältnis 2,7 zu 1 vermischt und mit einer Durchsatzgeschwindigkeit von 37,62 kg/h, bezogen auf das gesamte Reaktionsgemisch, in den Reaktor eingespeist. Die Verweilzeit in der ungekühlten Rohrstrecke zwischen Mischer 9 und Mikroreaktor beträgt 1,2 Sekunden. Dabei erwärmt sich das Gemisch von 20°C auf 96°C. Im ersten Mikrostruktur-Wärmeübertrager 10a wird das Reaktionsgemisch auf 76°C abgekühlt, erwärmt sich auf der Rohrstrecke 13b auf 123°C und wird im Wärmeübertrager 10b auf 57°C abgekühlt. Die hydrodynamische Verweilzeit zwischen Mischdüse 9 und dem zweiten Mikrostruktur-Wärmeübertrager 10b beträgt 14 Sekunden. Die Eintrittstemperatur des Kühlwassers in den ersten Mikrostruktur-Wärmeübertrager 10a beträgt 24,4°C, die Austrittstemperatur 51°C bei einer Durchsatzgeschwindigkeit von 80,3 kg/h. die Eintrittstemperatur des Kühlwassers in den zweiten Mikrostruktur-Wärmeübertrager 10b beträgt 24,4°C, die Austrittstemperatur 70°C bei einer Durchsatzgeschwindigkeit von 42,5 kg/h. Anschließend wird der Produktstrom ohne weitere Temperaturerhöhung in die Produktvorlage geleitet.

Die auf diesem Weg erzielte Produktausbeute bezogen auf eingesetztes Disulfid beträgt 88,2 % der Theorie.

Der Vorteil der Reaktionsführung mit zwei in Serie geschalteten Wärmeübertragern gegenüber der Reaktionsführung mit einem Wärmeübertrager liegt in der höheren Ausbeute an Disulfon bezogen sowohl auf Disulfid, als auch auf Wasserstoffperoxid und in der bei dieser Vorgehensweise realisierbaren höhere Eduktkonzentration, die zu einer höheren Raum-Zeitausbeute führt.

**Literatur**

[1] M. Bollyn et. al.: Chemical Plants and Processing 1(1996)

[2] K. R. Westerterp; E. J. Molga; K. B. Van Gelder:
Catalytic Hydrogenation Reactors for the Fine Chemicals Industries. Their Design and Operation, Chemical Engineering and Processing 36 (1997) 17- 27

[3] DE 37 09 278 C2

[4] US 4 516 632

[5] Microsystem Technology for Chemical and Biological Microreactors Workshop on Microsystem Technology, 20.-21. Februar 1995, Mainz,
Deutschland
Dechema-Monographs, Vol. 132
VCH Verlag, Weinheim, Deutschland, 1996

**Patentansprüche**

1. Verfahren zur Oxidation organischer Verbindungen in flüssiger Phase, bei dem die organische Verbindung in Form einer Lösung durch Zugabe eines peroxidischen, mindestens zwei verbundene Sauerstoffatome (-O - O-) enthaltenden Oxidationsmittels aufoxidiert wird, dadurch gekennzeichnet, daß ein die organische Verbindung enthaltender Eduktstrom A und ein das peroxidische Oxidationsmittel enthaltender Edukstrom B kontinuierlich miteinander vermischt werden und das flüssige Reaktionsgemisch einem Mikroreaktor (1) mit einer Schar von parallelen Reaktionskanälen (4') und benachbarten Kühlkanälen (3') zugeführt und dabei auf die Reaktionskanäle (4') verteilt wird und daß die Kühlkanäle (3') gleichzeitig mit einem Kühlmittel beschickt werden, um die durch die exotherme Oxidationsreaktion in den Reaktionskanälen (4') erzeugte Wärme im Mikroreaktor abzuführen, wobei die größte Kanalabmessung a der Reaktionskanäle senkrecht zur Strömungsrichtung der benachbarten Kanäle <1000 μm, vorzugsweise <500 μm beträgt und die Kleinste Wandstärke b zwischen den Reaktionskanälen und den Kühlkanälen <1000 μm, vorzugsweise <100 μm ist.

2. Verfahren nach Anspruch 1, dadurch gekennzeichnet, daß ein Mikroreaktor verwendet wird, dessen Reaktionskanäle (4') einen hydraulischen Kanaldurchmesser d <1000 μm, vorzugsweise <500 μm aufweisen.

3. Verfahren nach Anspruch 1 oder 2, dadurch gekennzeichnet, daß als Edukt A ein Olefin zur oxidativen Spaltung, ein Keton zur Umsetzung nach BaeyerVilliger oder ein Thiol zur Oxidation verwendet wird.

4. Verfahren nach Anspruch 1 - 2, dadurch gekennzeichnet, daß die zu oxidierende organische Verbindung aus einem organischen Sulfid besteht.

5. Verfahren nach Anspruch 4, dadurch gekennzeichnet, daß das organische Sulfid aus 3,5-Dithio-heptane-1,7-diol-diacetat besteht.

6. Verfahren nach Anspruch 1 - 5, dadurch gekennzeichnet, daß das peroxidische Oxidationsmittel aus Wasserstoffperoxid besteht.

7. Verfahren nach Anspruch 1 - 6, dadurch gekennzeichnet, daß zur Erzeugung des die organische Verbindung und das peroxidische Oxidationsmittel enthaltenden Reaktionsgemisches ein Düsenmischer (9) verwendet wird, bei dem das eine Edukt in das andere eingedüst wird.

8. Verfahren nach Anspruch 1 - 7, dadurch gekennzeichnet, daß das Reaktionsgemisch durch einen dem Mikroreaktor (1) vorgeschalteten Rohrreaktor (13a) mit einer Verweilzeit von 10 ms bis 20s, vorzugsweise von 10 ms bis 10 s, gefördert wird.

9. Verfahren nach Anspruch 8, dadurch gekennzeichnet, daß die Reaktion im Rohrreaktor (13a) unter adiabatischen Bedingungen durchgeführt wird und das heiße Reaktionsgemisch im Mikroreaktor (1) innerhalb von 1 ms bis 10 s, vorzugsweise innerhalb von 1 ms bis 1 s, um mehr als 20 °C heruntergekühlt wird.

10. Verfahren nach Anspruch 1 - 9, dadurch gekennzeichnet, daß das Reaktionsgemisch durch mehrere seriell hintereinander geschaltete Mikroreaktoren (10a ...10n) gefördert wird.

11. Verfahren nach Anspruch 10, dadurch gekennzeichnet, daß der Reaktionskanalquerschnitt der Mikroreaktoren (10a ... 10n) in Strömungsrichtung zunimmt.

12. Verfahren nach Anspruch 1 - 10, dadurch gekennzeichnet, daß zwischen den Mikroreaktoren (10a ... 10n) oder zwischen dem Düsenmischer (9) und dem Mikroreaktor (10a) an einer oder mehreren Stellen Edukte A, B oder im Kreislauf geführtes Reaktionsgemisch eingespeist werden.

13. Vorrichtung, insbesondere zur Durchführung des Verfahrens nach Anspruch 1 - 12, bestehend aus einem Mischer (9) zur kontinuierlichen Vermischung mindestens zweier Eduktströme A,B und einem nachgeschalteten Mikroreaktor (1) mit Reaktionskanälen (4') und Kühlkanälen (3'), wobei die größte Kanalabmessung a der Reaktionskanäle senkrecht zur Strömungsrichtung der benachbarten Kanäle <1000 µm, vorzugsweise <500 µm beträgt und die Kleinste Wandstärke b zwischen den Reaktionskanälen und den Kühlkanälen <1000 µm, vorzugsweise <100 µm ist, dadurch gekennzeichnet, daß der Mikroreaktor (1) seriell in mindestens zwei Miktoreaktorstufen (10a ... 10n) unterteilt ist, deren Reaktionskanalquerschnitte in Strömungsrichtung stufenweise zunehmen.

14. Vorrichtung nach Anspruch 13, dadurch gekennzeichnet, daß die Reaktionskanäle (4') einen hydraulischen Kanaldurchmesser d <1000 µm, vorzugsweise <500 µm aufweisen.

15. Vorrichtung nach Anspruch 13 - 14, dadurch gekennzeichnet, daß eine Kreislaufführung für das Reaktionsgemisch oder die Zufuhr von frischem Edukt zwischen einer oder mehreren Mikroreaktorstufen (10a ... 10n) vorgesehen ist.

16. Vorrichtung nach Anspruch 13 - 15, dadurch gekennzeichnet, daß zwischen dem Mischer (9) und den Mikroreaktorstufen (10a ... 10n) ein adiabatischer Rohrreaktor (13a) angeordnet ist.

# Fig. 1

EP 0 903 174 A1

# Fig. 2

Fig. 3

Komponente
$\Delta p_a \cdot V_a$

6a

Komponente
$\Delta p_b \cdot V_b$

6b

9

Mischung
zum Mikroreaktor 10

EP 0 903 174 A1

# Fig. 4a

6a

6m

9

10a 13b  10b  10c  13(n-2) 10(n-2)  10(n-1)  10n  13(n+1)

11a  11b  11c  11(n-2)  11(n-1)  11n

14c

15(n+1)/(n-2)

12

n vorzugsweise <20

Fig. 4b

EP 0 903 174 A1

# Fig. 4c

EP 0 903 174 A1

Fig.5

EP 0 903 174 A1

EP 0 903 174 A1

# Fig. 6

Europäisches
Patentamt

**EUROPÄISCHER RECHERCHENBERICHT**

Nummer der Anmeldung

EP 98 11 7121

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | DE 195 41 266 A (BAYER AG & FORSCHUNGSZENTUM KARLSRUHE GMBH) 7. Mai 1997 * das ganze Dokument * --- | 1,2,7-16 | B01J19/00 C07B33/00 C07C67/42 C07C69/017 C07C315/02 |
| A | WO 95 30476 A (BAYER AKTIENGESELLSCHAFT & FORSCHUNGSZENTRUM KARLSRUHE GMBH) 16. November 1995 * das ganze Dokument * --- | 1,2,6-17 | |
| P,A | WO 98 33582 A (FORSCHUNGSZENTRUM KARLSRUHE GMBH & BAYER AG) 6. August 1998 * das ganze Dokument * --- | 1,2,7-16 | |
| D,A | WO 88 06941 A (KERNFORSCHUNGSZENTRUM KARLSRUHE GMBH & MESSERSCHMIDT-BÖLKOW-BLOHM) 22. September 1988 * das ganze Dokument * ----- | | |

| | RECHERCHIERTE SACHGEBIETE (Int.Cl.6) |
|---|---|
| | B01J C07B C07C |

Der vorliegende Recherchenbericht wurde für alle Patentansprüche erstellt

| Recherchenort | Abschlußdatum der Recherche | Prüfer |
|---|---|---|
| DEN HAAG | 13. Januar 1999 | Stevnsborg, N |

KATEGORIE DER GENANNTEN DOKUMENTE

X : von besonderer Bedeutung allein betrachtet
Y : von besonderer Bedeutung in Verbindung mit einer
     anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
     nach dem Anmeldedatum veröffentlicht worden ist
D : in der Anmeldung angeführtes Dokument
L : aus anderen Gründen angeführtes Dokument
...........................................................................
& : Mitglied der gleichen Patentfamilie,übereinstimmendes
     Dokument

EPO FORM 1503 03.82 (P04C03)

**EP 0 903 174 A1**

**ANHANG ZUM EUROPÄISCHEN RECHERCHENBERICHT**
**ÜBER DIE EUROPÄISCHE PATENTANMELDUNG NR.**   EP 98 11 7121

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-01-1999

| Im Recherchenbericht angeführtes Patentdokument | Datum der Veröffentlichung | Mitglied(er) der Patentfamilie | | Datum der Veröffentlichung |
|---|---|---|---|---|
| DE 19541266 A | 07-05-1997 | CA | 2236666 A | 15-05-1997 |
| | | WO | 9717130 A | 15-05-1997 |
| | | EP | 0859660 A | 26-08-1998 |
| WO 9530476 A | 16-11-1995 | DE | 4416343 A | 16-11-1995 |
| | | DE | 4433439 A | 21-03-1996 |
| | | AT | 163568 T | 15-03-1998 |
| | | AT | 170773 T | 15-09-1998 |
| | | CA | 2189783 A | 16-11-1995 |
| | | DE | 59501565 D | 09-04-1998 |
| | | DE | 59503529 D | 15-10-1998 |
| | | DK | 758917 T | 30-03-1998 |
| | | WO | 9530475 A | 16-11-1995 |
| | | EP | 0758917 A | 26-02-1997 |
| | | EP | 0758918 A | 26-02-1997 |
| | | ES | 2113184 T | 16-04-1998 |
| | | ES | 2120204 T | 16-10-1998 |
| | | FI | 964483 A | 08-11-1996 |
| | | JP | 9506034 T | 17-06-1997 |
| | | JP | 9512742 T | 22-12-1997 |
| | | US | 5803600 A | 08-09-1998 |
| WO 9833582 A | 06-08-1998 | DE | 19703779 A | 13-08-1998 |
| WO 8806941 A | 22-09-1988 | DE | 3709278 A | 29-09-1988 |
| | | EP | 0391895 A | 17-10-1990 |
| | | JP | 3500861 T | 28-02-1991 |
| | | US | 5152060 A | 06-10-1992 |
| | | US | 5249359 A | 05-10-1993 |

EPO FORM P0461

Für nähere Einzelheiten zu diesem Anhang : siehe Amtsblatt des Europäischen Patentamts, Nr. 12/82